(19) Europäisches Patentamt

European Patent Office

Office européen des brevets

(11) **EP 4 614 500 A1**

(12) **EUROPEAN PATENT APPLICATION**

(43) Date of publication:
**10.09.2025 Bulletin 2025/37**

(21) Application number: **24162096.2**

(22) Date of filing: **07.03.2024**

(51) International Patent Classification (IPC):
**G16B 15/30** (2019.01)    **G16C 20/50** (2019.01)
**G16C 20/70** (2019.01)    **G16C 20/64** (2019.01)

(52) Cooperative Patent Classification (CPC):
**G16B 15/30; G16C 20/50; G16C 20/64;
G16C 20/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Zuse-Institut Berlin (ZIB)
14195 Berlin (DE)**
(72) Inventors:
• **SECKER, Christopher
  13357 Berlin (DE)**
• **WEBER, Marcus
  12045 Berlin (DE)**
• **SCHÜTTE, Christof
  14532 Kleinmachnow (DE)**

(74) Representative: **Hertin und Partner
Rechts- und Patentanwälte PartG mbB
Kurfürstendamm 63
10707 Berlin (DE)**

(54) **DRUG CANDIDATES AS PARETO OPTIMA IN CHEMICAL SPACE**

(57) The invention relates to a computer implemented method for identifying one or more ligands of interest for a target molecule. The method comprises (a) Providing a library comprising a plurality of (in silico) ligand structures, (b) Calculating/generating one or more additional (in silico) ligand structure(s) based on and/or starting from the library, by employing a machine learning software (artificial intelligence), (c) Analyzing and/or evaluating one or more ligand structure(s) provided in a. and/or b. by determining one or more characteristics of said one or more ligand structure(s), comprising at least the binding characteristics of said one or more ligand structure(s) to a target molecule, and (d) Selecting one or more ligand structure(s) belonging to a Pareto set of the optimal one or more characteristics determined in c., (e) Repeating b.-c. at least once, thereby considering the library according to a. and/or the one or more ligand structure(s) belonging to the Pareto set determined in any previous iteration of c., thereby determining the Pareto set of ligand structures for the one or more ligands of interest.

## Description

The invention lies in the field of biochemistry, pharmacology, and computer-implemented methods.

The invention relates to a computer implemented method for identifying one or more ligands of interest for a target molecule, comprising (a) Providing a library comprising a plurality of (in silico) ligand structures, (b) Calculating/generating one or more additional (in silico) ligand structure(s) based on and/or starting from the library, by employing a machine learning software (artificial intelligence), (c) Analyzing and/or evaluating one or more ligand structure(s) provided in a. and/or b. by determining one or more characteristics of said one or more ligand structure(s), comprising at least the binding characteristics of said one or more ligand structure(s) to a target molecule, and (d) Selecting one or more ligand structure(s) belonging to a Pareto set of the optimal one or more characteristics determined in c., (e) Repeating b.-c. at least once, thereby considering the library according to a. and/or the one or more ligand structure(s) belonging to the Pareto set determined in any previous iteration of c., thereby determining the Pareto set of ligand structures for the one or more ligands of interest.

## BACKGROUND OF THE INVENTION

Virtual screening is a data-driven computational technique for drug design. In its most prominent form, structure-based virtual screening (SBVS), drug candidates or ligands, such as small molecules, are tested against the binding site of a larger target molecule to estimate the binding strength between these two molecules structures. This binding strength (binding affinity) can be measured by the ratio of the off-rate and the on-rate, or by the Gibbs binding free energy, wherein promising drug candidates have high to highest binding affinity or low to lowest (negative) Gibbs binding free energy. Thus, SBVS optimizes a functional (affinity/Gibbs energy) in a set of given drug-like molecules, e.g., comprised within a compound/ligand library. Typical SBVS software solves this kind of optimization problem over gigantic (billion-sized) ligand libraries by high performance computing [2], mostly using approximate Gibbs energy like functionals in order to reduce computational complexity.

In its present form, SBVS has three main disadvantages: (A) the functional to be optimized is not the full affinity but an approximation, (B) it measures only binding strength and ignores potential side effects, and (C) it does not explore the whole chemical space of drug-like molecules but just known ligand libraries. The whole drug-like chemical space contains a huge number (estimates say $10^{60}$ structures [18, 19]) of small molecular structures.

In past research in MATHEON and MATH+ [3, 5], computational approaches delivered very good approximations of the full binding affinity of possible drug molecules and showed how to incorporate side effects into drug design with the result of several patented pain relief drugs [15, 1] that have now been out-licensed and will be clinically evaluated.

The inventors previously demonstrated that this progress now allows to solve (A) and (B) in a seamlessly integrated fashion, wherein SBVS becomes a multi-objective optimization problem (MOOP) by complementing the binding affinity function with an additional consideration of side effects. The inventors were able to demonstrate (for the $\mu$-opioid receptor as target) how the resulting MOOP can be solved by finding the respective Pareto front of optimal drug-like molecules in large target-specific libraries, i.e., in subsets of the chemical space. Furthermore, it was shown that the new MO-SBVS strategy in fact yields new drug candidates with optimal compromise between good binding affinity and mild side effects [4].

However, the MO-SBVS strategy still suffers from the limitations of the original SBVS strategies, namely that it cannot explore the full chemical space (comprising all molecules that exist and that may be synthesized). Such methods for exploring the full chemical space for new ligands and small molecules binding to a target of interest are still missing, although bearing a promising source of yet unknown, but potentially useful pharmaceutical compounds.

## SUMMARY OF THE INVENTION

In light of the prior art the technical problem underlying the present invention is to provide alternative and/or improved means for exploring and analyzing the chemical space with regard to specific target molecules of interest to identify potentially new suitable binding partners thereof.

This problem is solved by the features of the independent claims. Preferred embodiments of the present invention are provided by the dependent claims.

The invention therefore relates to a computer implemented method for identifying (determining) one or more ligands of interest for a target molecule, comprising:

    a. Providing a library comprising a plurality of (in silico) ligand structures, preferably represented as or comprising SMILES strings, and preferably wherein the library comprises known ligand structures,

    b. Calculating/generating one or more additional (in silico) ligand structure(s), preferably represented as SMILES strings, based on and/or starting from the library (and/or any previously identified member of the Pareto set), by employing a machine learning (ML) software or algorithm (artificial intelligence; AI),

c. Analyzing one or more ligand structure(s) provided in a. and/or b. by determining one or more characteristics of said one or more ligand structure(s), comprising at least the binding characteristics of said one or more ligand structure(s) to a target molecule, and

d. Selecting one or more ligand structure(s), preferably represented as SMILES strings, representing or belonging to the Pareto set for the optimal one or more characteristics determined in c.,
optionally adding the one or more ligand structure(s) representing or belonging to the Pareto set either to the library of a. or providing said structures instead of the library in step a.,

e. Repeating steps b.-d. or a.-d. at least once, thereby considering the library according to step a. and/or the one or more ligand structure(s) representing or belonging to a Pareto set selected in any previous iteration of step d., thereby determining (identifying) the Pareto set of ligand structures, preferably represented as SMILES strings, for the one or more ligand(s) of interest for a target molecule.

In embodiments step c. comprises analyzing one or more ligand structure(s) (the chemical structures of a ligand), provided in a. and/or b. by determining one or more characteristics of said one or more ligand structure(s), comprising at least the binding characteristics of said one or more ligand structure(s) to a target molecule, thereby identifying any ligand belonging to the Pareto set for the optimal one or more characteristics (Pareto set of interest).

Preferably in embodiments the one or more characteristics are indicative of the suitability of a ligand to interact with a target molecule. In embodiments the one or more characteristics may be differently chosen to be optimal depending on the context of interaction between the ligand and the target. For example, depending on the context, e.g., the pH and/or hydrophobicity of the medium in which interaction is supposed to happen, different characteristics of a ligand may be considered to be optimal. In some embodiments simply a specific binding affinity (e.g., strong binding) at physiological conditions may be of interest. The combination of a Pareto strategy with machine learning/AI in preferred embodiments enables the consideration of more than one characteristic when determining the optimal ligand or group of ligands for a target molecule.

Prior art strategies for finding molecule binding partner *in silica*, such as structure-based virtual screening (SBVS) techniques, still suffer from limitations such as that the analysis of the entire chemical space, namely the entirety of possible chemical molecules is not possible yet. The inventors therefore developed new strategies for identifying full Pareto sets of optimal molecule binding partners for a target molecule in the entire chemical space.

The inventors considered generative models to explore existing large libraries of drug-like molecules, such as the REAL database (comprising approx. 11 billion compounds). It was entirely surprising that by using such databases as a training set, generative models can be trained to randomly create novel elements of the drug-like chemical space [14]. The inventors found that the resulting generative algorithms allow for AI-guided exploration of the chemical space [9, 13, 8], as is shown in more detail below.

The inventors hypothesized that a multi-objective SBVS strategy may be combined with a stochastic exploration of the chemical space for finding the Pareto set (or essential parts of it) of a target binding structure. Solving multi-objective optimization problems in large-scale spaces is still a difficult task, especially finding a Pareto set, e.g., for chemical structures with optimal target binding) without already knowing a member thereof. The present invention comprises algorithms similar to evolutionary multi-objective optimization methods but preferably uses artificial intelligence (AI) or machine learning-based methods for the generation of new chemical structures, wherein the AI or ML-models can preferably be trained to consider rules, e.g., certain properties of the chemical structures, for the exploration of the chemical space. The inventors analyzed the underlying mathematics of such approaches, which offer a new understanding of the possibilities of exploring and screening the chemical space, which have nor been described the art before.

One surprising advantage of the present invention is the possibility to combine different molecule-target docking procedures and multiple AI or ML-based generative models (samplers) into the overall algorithmic scheme with a rigorously formulated common mathematical framework, which increases the chance for identifying suitable drug-like molecules for new therapeutic applications and with less side-effects.

In embodiments the present method comprises the training of a machine learning (ML) algorithm or model, or an artificial intelligence (AI) on existing chemical structures, preferably represented as SMILES strings, to generate new chemical structures. Newly generated chemical structures may be structures that are already known, but not represented/present in the training data (or initial data from which the algorithm starts to generate new chemical structures) or that are unknown and generated entirely new (proposed for the first time). In embodiments, such newly generated chemical structures may subsequently be analyzed *in silica,* as well as chemically synthesized and/or tested regarding their binding properties to a target molecule of interest and/or their pharmaceutical properties. In preferred embodiments the trained machine learning (ML) algorithm or model, or artificial intelligence (AI) has been trained with SMILES strings to subsequently generate new chemical structures represented as SMILES strings. In other words, in embodiments the ML algorithm or model, or AI is trained to construct and/or propose new SMILES strings.

In embodiments the term 'considering' in step e. may refer to the analysis and/or evaluation, for example using mathematical and/or statistical calculations, algorithms or machine learning models or AI, preferably as described herein, of the library (the molecules therein) according to step a. and/or the one or more ligand structure(s) representing or belonging to a Pareto set selected in d., wherein the consideration preferably comprises the addition of the previously determined/selected members of the Pareto set to the library in a. and/or the use/provision of said members of the Pareto set in step a. instead of the (initial) library.

In embodiments, an optimal binding or interaction of a ligand to a target molecule is determined based on one or more binding characteristics, comprising binding affinity (e.g., free energy in kcal/mol; preferably proportional to the binding affinity/strength) and/or (negative) Gibbs binding free energy of a ligand to the target molecule.

In embodiments, the binding affinity is a predicted binding affinity or an experimentally determined binding affinity. In embodiments, the on one or more binding characteristics further comprise other desired properties in drug discovery.

In embodiments, the one or more characteristics (of interest) determined in step c. further comprise one or more of target molecule (receptor) configuration, bioavailability (e.g., water solubility, fat solubility, solvent solubility), synthesizability, molecular weight and logP (hydrophilicity). In embodiments also (potential/expected) side effects upon administration of the ligand to a subject (e.g., mammal or particularly human) may be a characteristic of interest to be determined. Such side (potential/expected) effects may in embodiments also be derived from/comprised within a separate library or the library provided in a.

In embodiments a machine learning (ML) software or ML model, ML algorithm or AI may be or may comprise a generative model or generative algorithm, a generative adversarial network (GAN) or a deep generative model (DGM).

For example, in some embodiments the present method comprises the generation of new chemical structures, e.g., represented as SMILES strings, by a trained ML algorithm/model or AI, whereupon the binding affinity of the generated/proposed new chemical structures (molecules) is analyzed and evaluated by an expert (e.g., in wet lab experiments or drug screenings) or *in silica* by computational analysis, an AI or ML algorithm/model. For example, in some of such embodiments, the AI/ML proposes a certain number of new chemical structures (b.), based on the library / data provided in a., which are subsequently analyzed and evaluated regarding their properties or characteristics, particularly regarding their one or more characteristics of interest (c.), such as their binding characteristics to a target molecule and further chemical properties, such as hydrophobicity, bioavailability and/or other pharmacological properties, either by an expert (computationally and/or in the wet lab) or by an ML algorithm or AI. Preferably feedback is provided back to the method (e.g., only preferred/advantageous molecules are returned and/or undesired molecules are returned as 'exclusion criterion', and/or an evaluation regarding the one or more characteristics of said ligand structure(s) and/or their suitability as ligand for a target molecule of interest) (d.), such that during the next repetition of the method (e.) chemical structures with undesired or disadvantageous properties or characteristics are less likely to be generated and/or such that the ML algorithm starts from a chemical structure with desired or advantageous properties, for example, if a member of the pareto set for optimal characteristics has been generated and identified.

Hence in embodiments the present method further comprises the step of analyzing and/or evaluating one or more characteristics of the one or more (in b.) generated ligand structure(s).

In embodiments, step d. comprises further the provision of exclusion criteria and/or feedback to the ML algorithm (ML software) /AI regarding the analyzed and/or evaluated one or more characteristics of the of the one or more (in b.) generated ligand structure(s). In such embodiments the ML /AI preferably considers said exclusion criteria and/or feedback during the repetition of the method (according to e.) when calculating/generating one or more additional (in silico) ligand structure(s) in b.

In this context, 'new SMILES strings' preferably refers to SMILES strings that are not represented/present in the training data (or initial data from which the algorithm starts to generate new chemical structures) or that have not been described before and are generated entirely new.

Such analysis may in embodiments be performed using *in silico* methods or software programs, or using wet lab methods, such as chemical synthesis and molecular binding assays. In embodiments said analysis comprises assessing a biding strength (to a target molecule), bioavailability (e.g., water solubility, fat solubility, solvent solubility), synthesizability, molecular weight and/or logP (hydrophilicity), etc.

In embodiments the results of the analysis and/or evaluation of the newly generated structures are provided as feedback, as rules and/or as amendments in code back to the trained ML algorithm/model or AI.

In embodiments such feedback may comprise an evaluation whether the generated/proposed structures have a desired characteristic or not. In embodiments such feedback may comprise an evaluation of the generated structures regarding their suitability as ligand for a target molecule of interest. In embodiments such feedback may also comprise providing back only the newly generated structures with a desired characteristic (removing the structures not having the desired characteristics, e.g., strong binding or desired hydrophobicity etc.). In embodiments only newly generated structures are provided back to the algorithm/model/AI after the analysis and/or evaluation that belong to the Pareto set of interest.

In embodiments after step d. of selecting one or more ligand structure(s), preferably represented as SMILES strings, representing or belonging to the Pareto set for the optimal one or more characteristics determined in c., the selected one or more ligand structure(s) representing or belonging to the Pareto set of interest are provided back to the library in step a. and/or to the ML algorithm/model or AI in step b., whereupon the method steps a.-d. or b.-d. are repeated at least once, preferably until the entire Pareto set (or a predetermined number thereof) is identified. Hence, in embodiments any member of the Pareto set identified in step c. is selected in step d. and returned to step a. to be either included in the library or to serve instead of the library as basis for the prediction of new molecule structures in b.

In embodiments the newly (e.g., in b.) generated ligand structure(s) may either added to the initial library (e.g., provided in a.) or may be used (e.g., provided in a.) instead of the initial library to generate new ligand structure(s) (e.g., in a further repetition of b.). In embodiments the set of ligand structure(s) from which learning and/or a (e.g., more focused) generating/calculating (e.g., in a further repetition of b.) of ligand structure(s) takes place can comprise or consist of molecules from the Pareto set and/or molecules generated and/or provided in previous iterations of the method steps. In some embodiments a small number (e.g., <100, preferably <50) of molecules not belonging to the Pareto set, are added to the molecules/library provided in a. (wherein the library comprises/consists of members of the Pareto set) used for generating new ligand structure(s) in b., for example, in order to increase the diversity of generated ligand structure(s) or to avoid "overfitting" the ML model during subsequent ligand structure generation.

In one non-limiting example the method according to the invention is performed, wherein in each repletion of the method steps (according to step e.) in step b. 100 chemical structures are newly generated, and after the analysis and/or evaluation in step c. only those structures belonging to the Pareto set are added back to the library or are provided instead of the library to the ML or KI in step a., e.g., 10, 20, 30, 40, 50, 60, 70, 80, 90, 100, 150, 200, 250, 300 structures etc., wherein the method is repeated until after the analysis and/or evaluation in step c. 100 structures are determined as belonging to the Pareto set of interest, such that no structures have to be removed from the 100 structures generated in step b.

In some embodiments in each repetition of step b. chemical structures are newly generated until a total of 100 newly generated structures is obtained, preferably which were initially not comprised within the library in a., and that can subsequently be analyzed and/or evaluated in c.

In embodiments a preset number of , e.g., 10, 20, 30, 40 ,50, 60, 70, 80, 90, 100, 150, 200 etc. optionally each $\pm$ 5, structures are newly generated in each repetition of step b., wherein if one or more members of the Pareto set have been generated and selected in any previous repetition of the method, those are reintroduced in step a. (either instead or in addition to the library) as basis for the next calculation/generation in b. and are incorporated into the group (count) of newly generated structures in b. such that if 2 members of the Pareto set have yet been identified only 10-2 (8), 20-2 (18), 30-2(28), 40-2 (38), 100-2 (98), etc. new structures/molecules are generated in step b. until no new molecules have to be generated in b., as all or a preset number of Pareto set members has been identified. In embodiments said already in c./d. identified and selected members of the Pareto set are used instead or in addition to the library of a. as a basis for the generation of further new structures until the entire Pareto set or a preliminary determined number of members of the Pareto set has been identified.

In other words, in embodiments in step b. a predetermined number of molecule structures is calculated/generated based on and/or starting from the library and/or any in a previous repetition (according to e.) of the method generated, identified and selected members of the Pareto set (molecule structure), wherein any identified and selected Pareto set member is returned to step a., such that the method may be repeated until in step c./d. only members of the Pareto set are identified and selected and the predetermined number of molecule structures comprises only members of the Pareto set, such that no new molecules need to be generated to obtain the full Pareto set, wherein the 'full' Pareto set comprises the predetermined number of molecule structures.

In embodiments any member of the Pareto set being identified is added/included again into the group of newly generated structures in step b., until no new structures have to be generated in b., as all molecule structures analyzed and/or evaluated in step c./d. are members of the Pareto set.

In embodiments where a preset total/maximal number of molecules to be generated is used, e.g., 50 or 100 newly generated structures in total, the method stops (is not repeated according to e.) when said preset total/maximal number, e.g., 50 or 100, respectively, of members of the Pareto set for the optimal binding characteristic have been identified and selected in c./d.

In embodiments the method according to the invention is repeated after receiving feedback regarding a set of newly generated structures.

Preferably, the method according to the invention is repeated until only structures belonging to the Pareto set are (newly/additionally) generated in b. In other words, in embodiments b.-d. are repeated according to e. until all structures additionally generated in b. (or in one or more previous iterations of b.), and preferably also the structures selected in d., belong to the Pareto set for the one or more optimal characteristics of the ligands. Namely, until each in b. additionally generated structure is a member of the Pareto set, such that in d. all compounds analyzed in c. are selected as belonging to the Pareto set.

In embodiments, in step d. one or more ligand structure(s) representing/belonging to the Pareto set generated from the library (provided in a.) are provided back to the method.

In embodiments, the ligand structures are selected from simplified molecular input line entry specification (SMILES) strings, smiles arbitrary target specification (SMARTS) strings, Wiswesser Line Notation (WLN), ROSDAL, SLN and InChI, or any variation thereof.

In embodiments, the machine learning software (artificial intelligence; AI) is trained in advance based on a library comprising known chemical structures, preferably pharmaceutical compounds, signaling pathway members and/or receptor (ant-)agonists.

In embodiments the machine learning software (artificial intelligence; AI) is trained after the provision of the library comprising a plurality of (in silico) ligand structure(s) in a. based on said library, preferably before the generation of new ligand structure(s) in b. In embodiments the training/leaning also considers/implements/uses exclusion criteria and/or feedback provided with respect to the previously (e.g., in previous iteration(s) of the method steps) generated ligand structure(s), e.g., regarding the one or more characteristics of said ligand structure(s) and/or their suitability as ligand for a target molecule of interest.

In embodiments, the ligand of interest is able to bind to an active center, a target binding site and/or an epitope binding site of the target molecule.

In embodiments, the ligand of interest is a small molecule compound and/or wherein the library provided in a. comprises one or more (drug-like) small molecule compounds.

In embodiments, a generated additional (in silico) ligand structure possesses a (pre-)defined chemical distance to the chemical structure it is generated from or to the previously generated ligand structure, such that said ligand structures differ in their chemical structures from each other by a (pre-)defined degree (the two subsequent ligand structures differ to a (pre-)defined degree with regard to their chemical structures). In other words, in embodiments initially the chemical distance between two subsequent structures in step b. is (pre-)define to be quite broad/far, such that the random walk initially results in structures being successively generated between which exists a large chemical distance (two subsequent ligand structures differ to a great(er) degree with regard to their chemical structures). Thus, in embodiments, initially during the random walk of the algorithm through chemical space ligand structures are generated that possess a large(r) chemical distance between each other. Thereby the diversity of generated and analyzed structures is increased such that the search spans a broad scope of different chemical structures. This preferably also reduces the time needed to find chemical structures that show more optimal binding characteristics, than if only similar structures are generated and analyzed initially. In embodiments, once structures with promising (near optimal) binding properties have been identified, the chemical distance between generated ligand structures may be reduced subsequently. For example, once one or more structures of the Pareto sets seem to have been identified (e.g., in c.) and preferably selected (e.g., in d.) during the random walk through chemical space (e.g., during b.) the algorithm preferably changes to exploring C with regard to (starting from a member) the Pareto set of chemical structures with optimal target binding (e.g., as said member(s) of the Pareto set are either added to the library in a. or are provided instead of the library in a.).

In embodiments, analyzing in c. comprises a stepwise/sequential analysis of ligand structures comprised within the ligand library and/or calculated/generated in b., and preferably also of interpolated chemical structures, wherein the subsequently analyzed structures are chosen randomly until an element of the Pareto set of the optimal binding structure to the target molecule is identified, wherefrom only chemical structures structurally similar, e.g., within a specific Tversky index, to said element of the Pareto set are selected from the library and/or calculated/generated in step b. and analyzed in step c. In embodiments herein, a specific Tversky index may stochastically be obtained from the machine learning model that contains the (Pareto set)-based (fine-tuned or adjusted) model weights.

In embodiments, the calculation/generation of the one or more additional ligand structure(s) in b. also comprises interpolation between known ligand structures from the library. For example, the interpolation may refer in embodiments to the generation, computation or (theoretical) consideration of a 'gradient' between chemical structures that are similar to each other (share a low chemical distance) for the generation of new chemical/ligand structures of existing or previously generated chemical/ligand structures. In exemplary embodiments and depending on the generative ML model used, the interpolation between known ligand structures can, e.g., be described as geometric interpolation in the latent space, for example for Variational Autoencoder (VAE) models or Generative Adversarial Networks (GAN). Examples of interpolation between chemical structures may also be derived from [21], [22] and [23].

In embodiments, determining one or more characteristics of said one or more ligand structure(s) in c. comprises the employment of one or more computational (algorithmic) calculations for the ligand structures calculated/generated in b., preferably performed or supervised by a subject.

In embodiments, the machine learning software comprises a generative model, a generative adversarial network (GAN) or a deep generative model (DGM).

In embodiments the method according to the present invention may be computer-implemented, thereby constituting in embodiments a computer-implemented method.

In another aspect the present invention relates to a software or computer program product for identifying one or more ligands of interest for a target molecule, wherein when said software or computer program product is executed the following steps are conducted:

a. Receiving data of a library comprising a plurality of (in silico) ligand structures,

b. Calculating/generating one or more additional (in silico) ligand structure(s) based on the library, by employing a machine learning software (artificial intelligence; AI),

c. Analyzing and/or evaluating one or more ligand structure(s) provided in a. and/or b. by determining one or more characteristics of said one or more ligand structure(s), comprising at least the binding characteristics of said one or more ligand structure(s) to a target molecule, and

d. Selecting one or more ligand structure(s) belonging to a Pareto set of the optimal one or more characteristics determined in c.,

e. Repeating b.-c. at least once, thereby considering the library according to a. and/or the one or more ligand structure(s) belonging to the Pareto set selected in any previous iteration of d., and thereby determining the Pareto set of ligand structures for the one or more ligands of interest,

f. optionally transmitting and displaying an output of the software or computer program product to a graphical user interface, preferably comprising the ligand structure(s) belonging to the Pareto set of ligand structures for the one or more ligands of interest.

In embodiments the present invention relates to a software or computer program product for identifying (determining) one or more ligands of interest for a target molecule, wherein when said software or computer program product is executed the steps of the (computer-implemented) method according to the invention are conducted.

In another aspect the present invention relates to a computer-readable storage device, comprising a software or computer program product according to the invention.

In another aspect the present invention relates to a computer-implemented method for producing a database of one or more ligands of interest for a target molecule, comprising

a. providing a library comprising a plurality of (in silico) ligand structures,

b. Calculating/generating one or more additional (in silico) ligand structure(s) based on the library, by employing a machine learning software (artificial intelligence; AI),

c. Analyzing and/or evaluating one or more ligand structure(s) provided in a. and/or b. by determining one or more characteristics of said one or more ligand structure(s), comprising at least the binding characteristics of said one or more ligand structure(s) to a target molecule, and

d. Selecting one or more ligand structure(s) belonging to a Pareto set of the optimal one or more characteristics determined in c.,

e. Repeating b.-c. at least once, thereby considering the library according to a. and/or the one or more ligand structure(s) belonging to the Pareto set selected in any previous iteration of d., thereby identifying (determining) the Pareto set of ligand structures for the one or more ligands of interest,

f. generating a database of one or more ligands belonging to the Pareto set, comprising for each respective ligand structure(s), one or more characteristics determined in c. comprising at least the binding characteristics of said one or more ligand structure(s) to a target molecule.

In embodiments the present invention relates to a computer-readable storage device, comprising the database constructed according to the method above.

Each optional or preferred feature of the invention that is disclosed or described in the context of one aspect of the invention is herewith also disclosed in the context of the other aspects of the invention described herein.

All features disclosed in the context of the (computer-implemented) method according to the invention also relate to the computer program or, and are herewith disclosed also in the context of, the data storage device and the method for constructing a database according to the invention in a subject, and vice versa. The various aspects of the invention are unified by, benefit from, are based on and/or are linked by the common and surprising finding of the improved generation/prediction of new ligand structures for a target molecule according to the present invention combining a Pareto strategy with machine learning/AI for the exploration of the chemical space to generate/predict new chemical structures.

## DETAILED DESCRIPTION OF THE INVENTION

All cited documents of the patent and non-patent literature are hereby incorporated by reference in their entirety.

The present invention is directed to a method for identifying one or more ligands of interest for a target molecule by determining from an initial library comprising molecule structures a Pareto set for the optimal characteristics of interest

In the context of the present invention the term 'ligand' refers to any chemical molecule that is able to bind to a target molecule of interest. A 'ligand structure' herein preferably refers to the chemical structure of a ligand. A 'target molecule' of interest may be, for example, another chemical molecule, a protein, a receptor protein, a protein complex, nucleic acid(s) or any combination thereof.

Herein, "nucleic acid" may preferably refer to DNA (deoxyribonucleic acid), gDNA (genomic deoxyribonucleic acid), RNA (ribonucleic acid), gRNA (genomic ribonucleic acid), mRNA (messenger ribonucleic acid), tRNA and cDNA (complementary deoxyribonucleic acid synthesized from RNA template), or any combination thereof.

Herein, a library refers to a data set or database comprising preferably more than one chemical structure. In embodiments the library may comprise a large plurality of diverse chemical molecule structures. In embodiments the library may comprise chemical structures of molecules belonging to a certain molecule type or group, to a certain (pharmaceutical) compound class, to a certain protein family, cellular receptor family, cellular signaling pathway, metabolic pathway, pharmaceutical and/or biochemical pathway etc. Non-limiting examples would be a library only comprising drug-like small molecules or molecules and analogous belonging to a certain drug-class (e.g., opioids, receptor inhibitors or antibiotics). The skilled person is aware of suitable starting libraries comprising a diverse range of chemical molecules and structures or a targeted/pre-selected kind of chemical molecules and structures. Libraries according to the invention may also comprise further information, e.g., regarding the one or more characteristics of interest such that an algorithm, model or AI may also learn from said library to associate a certain structure with a certain characteristic. In embodiments a training library used to train the algorithm, model or AI may be the library provided in a. or a different library or a combination thereof, e.g., comprising additional data (for the purpose of training) on the chemical structures used in the library provided in a.

The term 'SMILES' commonly refers to the simplified molecular-input line-entry system, which represents a linear (string) format for characterizing the structure of a chemical molecule. SMILES strings can also be converted back into two-dimensional or three-dimensional molecules models. Since their development in the 1980 [20], SMILES have been further improved by the Daylight Chemical Information Systems Inc. (https://daylight.com/smiles/).

The term 'SMARTS' SMILES commonly refers to arbitrary target specification and represents a linear (string) format for specifying the structure and substructural patterns of chemical molecules. SMARTS is an advanced form of SMILES that facilitates precise substructural specification.

'Wiswesser Line Notation' (WLN) was the first linear structure code for chemical compounds that is now considered obsolete due to its complexity.

'ROSDAL' refers in chemistry to Representation of Organic Structure Descriptions Arranged Linearly, which is a further linear (string) format for specifying the structure of chemical molecules that was developed by S. Welford, Jet al., in the 1980s that encodes chemical structures using alphanumeric symbols.

The 'SLN' or 'SYBYL' line notation further linear (string) format for specifying the structure of chemical molecules and provides full Markush, R-Group, reaction, and macro atom capabilities in a single string notation. SLN enables the representation of reactions, chemical/molecular structures, libraries of structures, molecular fragments and formulations.

'InChl' refers to UPAC International Chemical Identifier and is a linear (string) format for specifying the structure of chemical molecules that was developed by IUPAC and the InChl Trust.

'Drug like' molecules are molecules that are considered to comprise characteristics, that are desirable in a pharmaceutical drug, such as bioavailability, solubility (hydrophilicity or hydrophobicity etc.), molecular weight, target affinity etc. The drug likeliness of a compound/molecule may be determined based on the chemical/molecular structure.

The term 'chemical space' generally refers to the entirety of all chemical molecules that exist or can be synthesized [16-19]. The term is commonly used in the field of cheminformatics, which aims to explore all chemical molecules in the chemical space, particularly the potential pharmacologically active molecules whose total number is estimated to be around $10^{60}$ molecules. When referring to pharmacologically active molecules, the so-called Lipinski rules are commonly applied [18], which, for example, reduce the maximum molecular weight to 500 Da (thereby to max. 30 atoms) and to the elements carbon, hydrogen, oxygen, nitrogen and sulfur. The exploration of chemical space or walk through the chemical space preferably refers herein to the method step b. comprising generating additional/new ligand structures.

In general, the field of 'machine learning" (ML)' is commonly associated with the field of artificial intelligence (AI),

where machine learning generally refers to computational methods and models that "learn" from training data. For example, a machine learning method (or machine learning software) is able to construct models (ML models) from training data. By this, ML algorithms or models are able to independently (without being programmed to) make decisions and predictions, based on the knowledge learned from training data.

'Generative models' or 'generative algorithms' commonly refer to statistical models capable of generating new data, based on training data by approximating and generating a joint distribution of the target and the training data. In embodiments, generative models may be employed, besides the generation of new data, also for classification tasks. In embodiments generative models may be combined with neural networks to obtain deep generative models (DGMs). In embodiments besides or instead of generative models, generative adversarial networks (GANs) may be used, as they are capable of extended deep learning for more complex generation tasks and translation tasks across different data arrangements.

In the context of the present invention, 'training data' and/or the library (e.g., database/dataset) referred to in step a. preferably comprises a plurality or at least more than one chemical structure and/or SMILES strings and/or any other information on a chemical molecule suitable for predicting further chemical molecules (such as a library/database of chemical structures and/or SMILES strings of ligands known to bind to a certain family of proteins or a database/library comprising chemical structures and/or SMILES strings of any pharmacological active molecule). In embodiments training data and/or a library according to step a. comprises further information on the molecules comprised therein, preferably also information relating to one or more characteristics, e.g., the binding characteristics, target molecule (receptor) configuration, bioavailability (e.g., water solubility, fat solubility, solvent solubility and/or the fraction of a substance/pharmacological compound that reaches the systemic circulation of a patient after administration), synthesizability, molecular weight and logP (hydrophilicity), of the comprised molecules. In embodiments also predicted side effects upon administration of the ligand to a subject (e.g., mammal or particularly human) may be a characteristic of interest to be analyzed. In embodiments the machine learning model/software is trained in advance based on a library comprising at least one or more known chemical structures, preferably pharmaceutical compounds, signaling pathway members (cellular signaling proteins) and/or receptor (ant-) agonists.

In embodiments a 'receptor configuration' may refer to a certain three dimensional shape of a receptor protein. As receptors are dynamic protein molecules able of small and rapid structural fluctuations, one receptor protein may comprise different configurations, e.g., when present in a receptor complex or certain biological environment where the shape of a single receptor may be influenced by surrounding molecules and proteins through electrostatic interactions, e.g., Van der Waals forces and hydrogen bonds.

In general, a Pareto optimality or Pareto optimal solution may be defined as a set of solutions wherein one solution cannot be further changed or improved without changing least one other solution within the set for the worse. In other words, a Pareto optimal solution described a solution which cannot be improved for one property of interest without having to worsen another at the same time. For example, a Pareto optimal of a ligand structure may be regarded as a structure wherein no change could lead to an improvement of one characteristic without the worsening another characteristic, namely such that no change would lead to a further overall improvement. In general, a Pareto set is the set of all Pareto optimal situations. For example, in embodiments a ligand structure for a target is generated, wherein strong affinity to the target and weak affinity to off-targets or a high bioavailability are both desired, at a certain point a structure can no longer be modified to increase the binding affinity to the target without also increasing the binding also to off targets or reducing the bioavailability. One advantage of a Pareto optimization is that it allows for the consideration of trade-offs (improve one aspect but worsen another) between objectives.

Commonly, structure-based virtual screening (SBVS) is a method for testing chemical molecules ("drug candidates" or "ligands"), e.g., small molecules for their binding to a target-binding site of a larger molecule ("target"), e.g., a protein, protein complex or receptor, to estimate the binding strength between said two molecules ("structures"). The estimated/determined binding strength ("affinity", that may be considered a "binding characteristic") can be measured, for example, by the Gibbs binding free energy. The binding strength of a ligand to a target may also be measured by the ratio of the 'off-rate' ($K_{off}$ [$S^{-1}$]; dissociation rate) and the 'on-rate' ($K_{on}$ [$M^{-1}S^{-1}$]; association rate) of a ligand binding to a target. In general, the binding strength is dependent on intermolecular forces, e.g., hydrogen bonds, ionic bonds and Van der Waals forces, wherein a binding is actually reversible (in most biological systems) through dissociation. In such examples, promising ('good') ligand/drug candidates have high to highest binding affinity (resulting from greater attractive forces between the ligand and its target) or low to lowest (negative) Gibbs binding free energy. The binding affinity of a ligand to a target can be described as a dissociation constant ($K_D$ [M]). Common methods for determining dissociation constants ($K_d$) comprise isothermal titration calorimetry, surface plasmon resonance or fluorescence quenching methods. A ligand's binding affinity can also be characterized indirectly by the IC50 value. The IC50 may be determined in a competitive binding reaction of the ligand, a reference ligand and a target, wherein the IC50 value is the concentration of a ligand required to displace 50% of a preset concentration of reference ligand. From the IC50 the $K_i$ of a ligand may be determined through the Cheng Prusoff equation.

A specific form of ligand-target binding is the binding of a ligand to the binding site of a receptor. In such embodiments ligands may be, e.g., agonists, antagonists, partial- or co-agonists, wherein an agonist induces a downstream effect upon binding to the receptor, while the binding of an antagonist would not induce any downstream response (blocking). In embodiments the binding of the agonist to the receptor may induce the activation of a receptor. However, the binding of the agonist to the receptor may be either a stable or transient event, of either strong or weak interaction, such that the receptor is activated preferably by agonist-receptor physical interaction.

The skilled person is familiar with suitable assays for testing the binding affinity of a ligand to a target or the pharmacological activity of a compound. For example, a molecule with potential pharmacological properties may be incubated with a cell or administered to an animal model following an appropriate analysis of the pharmacological effects of said administration, e.g., against a certain disease or representative cells thereof. Common assays for testing the pharmacological activity of a compound are *in vitro* (high-throughput) drug screenings that analyze the effects of a drug on certain cells *in vitro.* Alternatively or additionally *in vivo* studies in model animals or xenografts can be used to test the pharmacological activity of a compound.

**FIGURES**

The invention is further described by the following figures. These are not intended to limit the scope of the invention but represent preferred embodiments of aspects of the invention provided for greater illustration of the invention described herein.

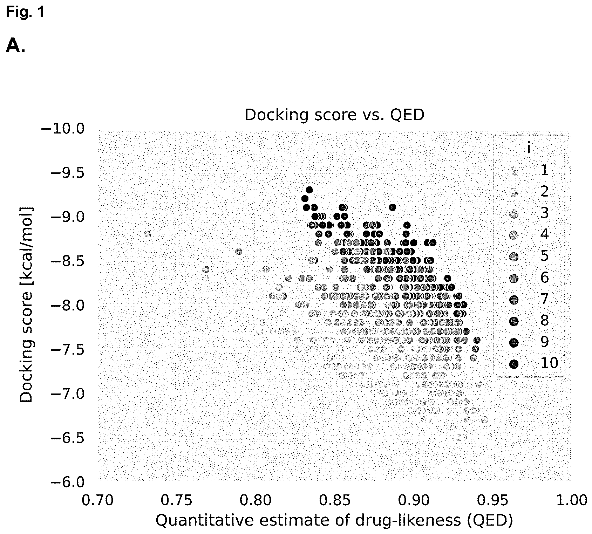

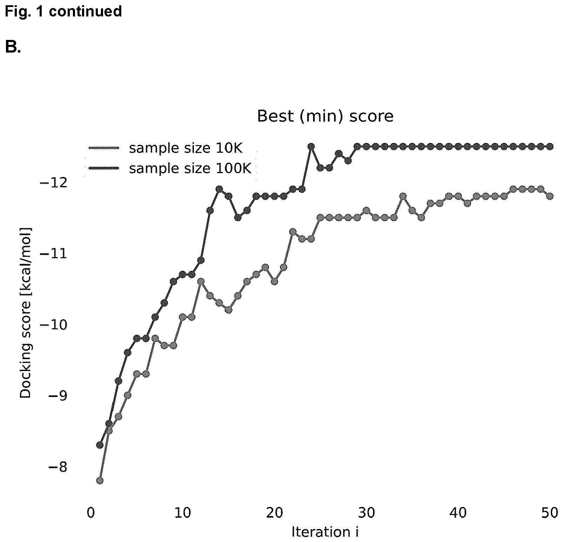

**Figure 1:** The figure depicts one exemplary embodiment of the present invention. A. The figure shows the iterative exploration of chemical space by the sets $B_i$ and improvement of the two-objective functional given by drug-likeliness and docking score (=free energy in kcal/mol, maximal absolute value is best); the progressing iteration index $i = 1, ... , 10$ is indicated by darker and darker colors. B. The figure shows the evolution of the best docking score in the set $A_i$ during the iteration for two different sample sizes of the sets $A_i$ (sample size of 10K shown in light grey, bottom line; sample size 100K black line, on top of light grey line in graph).

**Figure 2:** Virtual screening workflow for identifying pH-specific (as characteristic) MOR agonists (MOR target molecule ligands).

**Figure 3:** Identifying pH-specific MOR agonists from the Enamine library (all ligands; dark grey). The most promising drug candidates (that optimally combine the two conflict objectives) are found within the Pareto set (light grey data points).

**Figure 4:** Workflow to pre-train the molecular generative model with molecules from the ChEMBL database.

**Figure 5:** Workflow to refine the pre-trained model with candidate molecules within the Pareto set. The molecular structure of the top three Enamine drug candidates from the Pareto set (light grey data points) that showed the highest pH-specificity for the MOR are shown.

**Figure 6:** The iterative cycle conducted to identify drug candidates with improved pH-specificity for the MOR. The generative machine learning model is iteratively refined with the screening hits in the Pareto set (light grey data points).

**Figure 7:** Results from the application of the method to the identification of optimized pH-specific MOR agonists. The upper left panel (i = 0) shows the screening results of the 50.992 molecules from the Enamine library. The panels to the right show the screening results of the ~3.000 molecules obtained from the refined machine learning models after one (i = 1), five (i = 5; Fig. 7 cont.) or nine (i = 9; Fig. 7 cont.) Pareto set refinement cycles. The lower panel shows the data points of the top 100 pH-specific drug candidates from the respective iteration cycles.

**EXAMPLES**

The invention is further described by the following examples. These are not intended to limit the scope of the invention but represent preferred embodiments of aspects of the invention provided for greater illustration of the invention described herein.

**Example 1**

When analyzing suitable approaches for the present method the inventors combined the approaches of

(I) an analysis of the mathematical properties of a 'random walk' in chemical space (a screening through the entirety of possible chemical structures, wherein each next structure to be analyzed is chosen (generated) randomly) by a generative algorithm,

(II) construction of an algorithmic scheme that explores chemical space while improving the multi-objective functional, and description of its convergence properties to the Pareto set of a target binding structure,

(III) efficient implementation of the algorithm and application to real-world drug design problems.

For step (I) the inventors considered generative algorithms that define a random walk in the chemical space 'C', wherein their properties depend on a parameter vector p that can be tuned to allow for "larger" or "smaller" steps from one chemical structure to the next in the chemical space 'C', wherein larger and smaller steps are measured respectively in the expected "structural distance" between one chemical structure (from which the algorithm moves to the next structure) and the subsequently generated chemical structure. The "structural distance" between two subsequently generated structures is preferably measured by established chemical similarity indices, like the Tversky index. However, the investors aimed to improve such a strategy by introducing an adequate metric in C. Recent approaches to this problem like in [11] or via stochiometric distances proved inappropriate for the task and wherein a "step metric" preferably links the "structural distance" (between a prior and a subsequently generated chemical structure) to the expected "step size" depending on the parameter vector p. In embodiments a continuous version of C is constructed, e.g., by interpolation between chemical structures like in molecular alchemy.

For step (II), to define the concept of "improving the functional", the multi-objective functional is denoted by $F : C \rightarrow Rm$, and $x \in A \subset C$ non-dominated in A if there is no $y \in A$ such that $F(x) < F(y)$, where the last relation means that $F(x) \leq F(y)$ for all components i of F and $F(x) \neq F(y)$.

On this background, the exploration algorithm could be of the general following form, similar to evolutionary multi-objective optimization: Starting from a known compound library $A0 \subset C$ and set $i = 0$, steps (a)-(c) are repeated iteratively, wherein (a) comprises computing F for all $x \in A$, (b) comprises finding the non-dominated structures in A (or an appropriate relaxation), and call this subset B, and (c) comprises performing a random walk starting from set B to randomly generate a set $A+1$ of new structures, set $i = i + 1$. Preferably, whether and how this kind of algorithm converges to (parts of) the Pareto set depends on the properties of the random walk.

In embodiments initially parameters may be used for p, such that the random walk initially results in structures being successively generated between which exists a large chemical distance. In other words, in embodiments, initially during the random walk of the algorithm through chemical space chemical structures are generated that possess a large chemical distance between each other (that differ significantly with regard to their chemical structures). Thereby the diversity of generated and analyzed structures is increased such that the search spans a broad scope of different chemical structures. This preferably also reduces the time needed to find chemical structures that show more optimal binding characteristics, than if only similar structures are generated and analyzed initially. In embodiments, once structures with promising (near optimal) binding properties have been identified, the distances between chemical structures generated may be reduced subsequently. For example, once one or more structures of the Pareto sets seem to have been identified during the random walk through chemical space the algorithm preferably changes to exploring C with regard to (starting from a member) the Pareto set of chemical structures with optimal target binding.

One non-limiting example of such an embodiment is illustrated in Figure 1, wherein the left subfigure shows how the sets B iteratively improve the two-objective functional, while the right subfigure shows the improvement of one of the objectives (docking score) within the candidate sets A during the iteration and indicates that larger sets A may perform better.

Given that the chemical space contains an order of $10^{60}$ elements, in cases where the inventors expect that the Pareto set would be too large to be completely represented (e.g., due to lack of computation capacity/resources/time), convergence may not be measured in terms of "covering the Pareto set", but in terms of the convergence of the parameters which define the AI-based generative model in the sense that towards the end, the AI-based algorithm proposes only structures belonging to or being close to the Pareto set.

For step (III), the inventors implemented a large scale Pareto Screening Framework within a high-throughput SBVS platform (e.g., Virtual Flow [2]). This allowed for an implementation of the developed method for state of the art applications, by enabling computationally most expensive tasks (such as computing the 'docking score') being outsourced to high-performance computation facilities. In a final step the developed algorithm was adapted to given pharmaceutical problems.

**Example 2**

Compound screening by a Pareto set in chemical space to identify pH-specific MOR agonists

The present example provides one embodiment for performing the present method. Herein, a compound screening using a Pareto set in chemical space can be used to identify drug candidates that strongly bind to the $\mu$-opioid receptor (MOR) at acidic pH, but less strong to the MOR at neutral pH. This is one aim of the present example since pH-specific binding to the MOR can reduce side effects of opioids (https://www.science.org/doi/10.1126/science.aai8636).
One embodiment of the present method to perform compound screening by a Pareto set in chemical space was performed to identify pH-specific MOR drug candidates with the following steps:

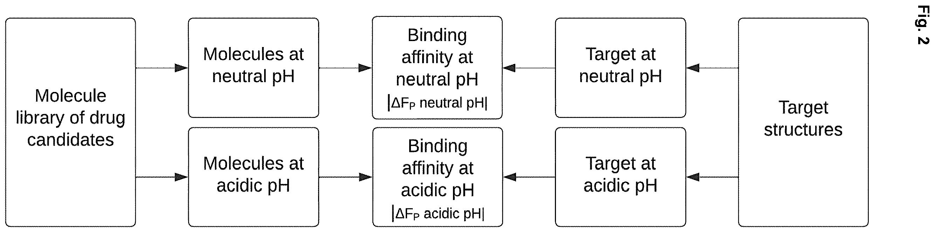

1. A set of known drug candidates from the Enamine library (https://enamine.net/) were tested for their potential binding affinity to two different target structures: (1) to the MOR structure in a neutral (pH=7.4) environment, and (2) to the MOR structure in an acidic (pH=5.0) environment (Figure 2). For the prediction of binding affinities (binding free energies, $|\Delta F_P|$), the molecular docking workflow engine Virtual Flow (https://github.com/VirtualFlow) was used to screen 50.992 drug candidates against the two targets on a high-performance computing cluster.

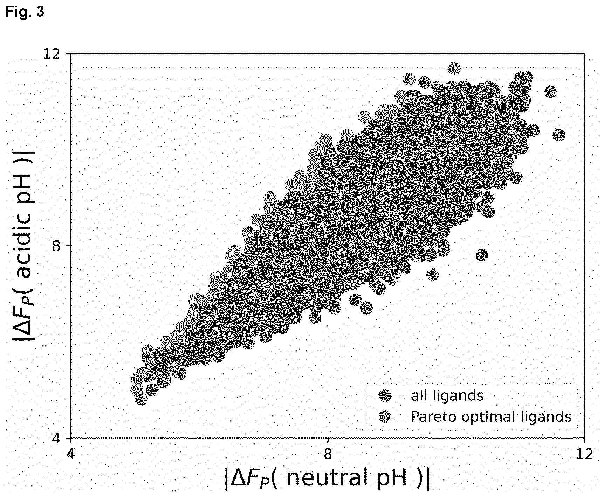

2. A set of Pareto-optimal drug candidates, i.e. molecules with a high binding affinity at acidic pH ($|\Delta F_P(\text{acidic pH})|$) and a low binding affinity at neutral pH ($|\Delta F_P(\text{neutral pH})|$), were extracted from the molecular docking results (Figure 3, light grey data points).
Extraction of the Pareto-optimal candidates was achieved as outlined by the following pseudo code:

```
sort data_points by increasing x_value
    save as sorted_data_points
    set maximum_y_value to -infinity
    # Iterate through sorted data points
    for data_point in sorted_data_points:
        # If y_value is greater than maximum_y_value,
        # add the point to the Pareto set and update maximum_y_value
      if data_point_y_value >= maximum_y_value:
             add data_point to Pareto_set
             set maximum_y_value = data_point_y_value
```

Pareto-optimal candidates were also extracted by ranking the virtual screening hits in descending order using the following scoring metric of the min-max normalized predicted binding affinities $|\Delta F_P(\text{neutral pH})|$ and $|\Delta F_P(\text{acidic pH})|$, respectively:

$$\text{score} = \lambda * |\Delta F_{P\text{norm}}(\text{acidic pH})| + (1 - \lambda) * (1 - |\Delta F_{P\text{norm}}(\text{neutral pH})|)$$

with $\lambda \in \{0.3, 0.4, 0.5, 0.6, 0.7\}$

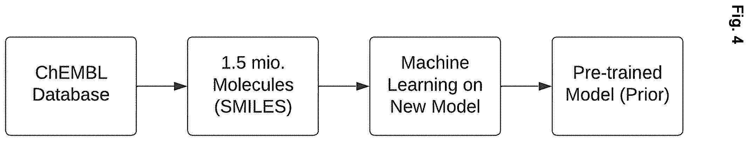

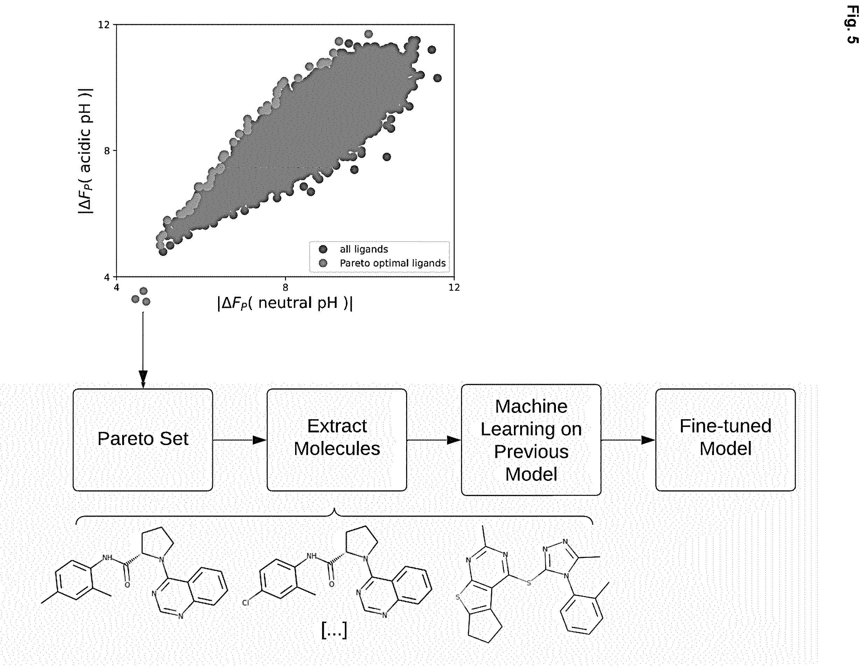

3. A molecular generative machine learning model (https://github.com/undeadpixel/reinvent-randomized) was first trained with a chemically diverse library of ~1.5 mio. known drug-like molecules from the ChEMBL Database (https://www.ebi.ac.uk/chembl/; Figure 4).
The resulting pre-trained machine learning model (prior) was then fine-tuned by performing additional training epochs on the prior with molecules from the Pareto-optimal drug candidates extracted in step 2 (Figure 5).

4. The fine-tuned machine learning model was then used to randomly generate ~3.000 novel molecules based on the learned model weights.

5. The generated compounds were again evaluated for their binding to the two target structures, i.e. the MOR in a neutral environment and the MOR in an acidic environment (as in step 1).

6. From the novel data set, the Pareto-optimal drug candidates are extracted (as in step 2).

7. Extracted drug candidates are used to further refine the fine-tuned machine learning model from step 3 or the previous iteration, respectively.

8. The steps 3 to 7 were iteratively repeated to identify optimized drug candidates with pH-specific binding to the MOR.

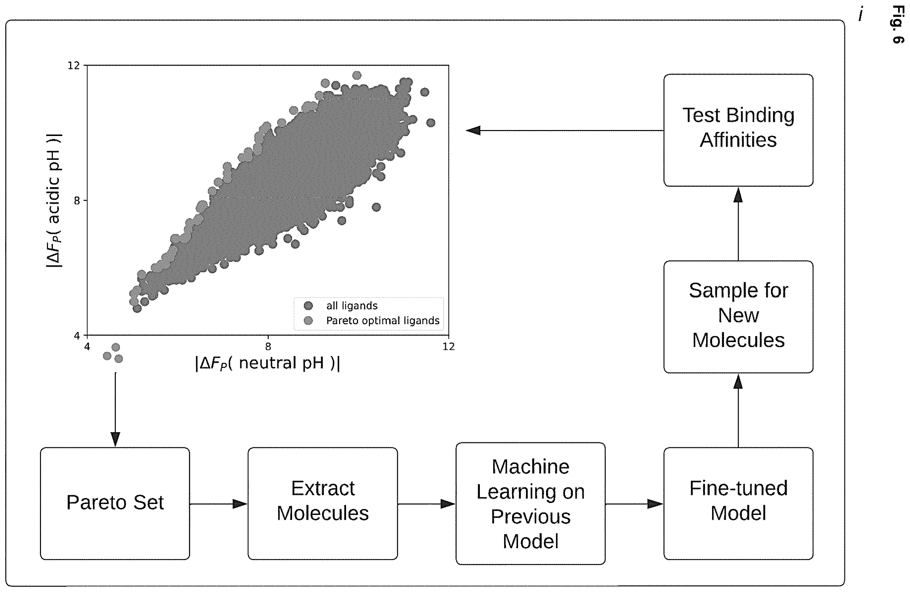

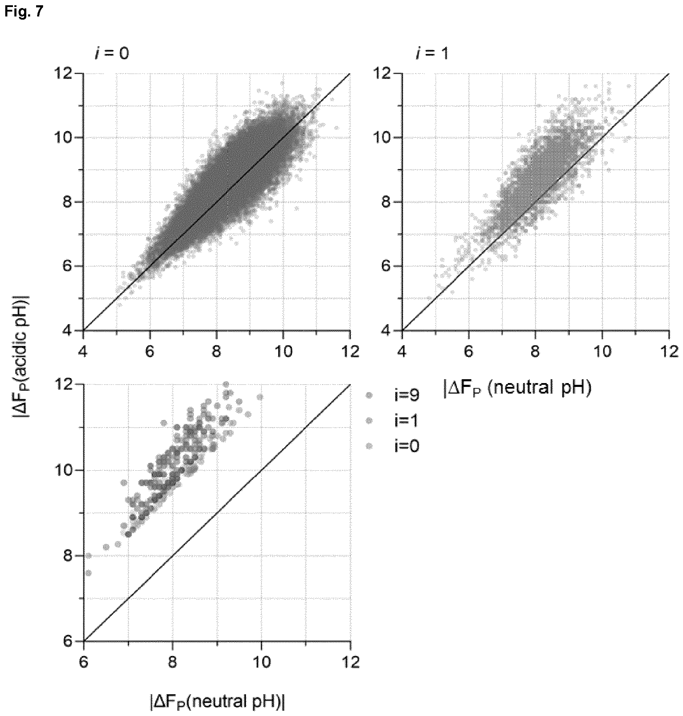

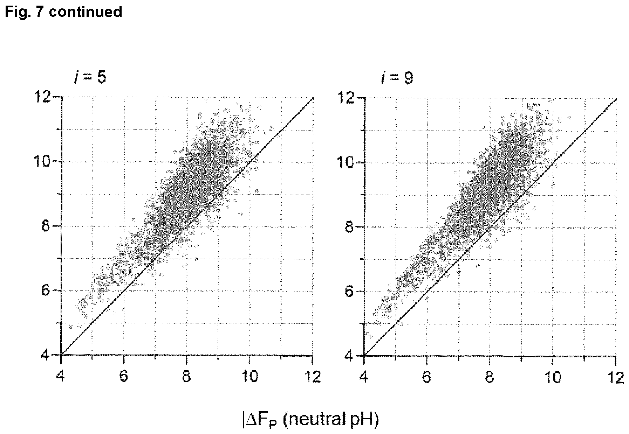

Iteratively refining the machine learning model over multiple iterations lead to the generation and identification of drug candidates with improved pH-specificity for the MOR (Figure 7). The data set at i = 0 represents the screening results from the previously known drug candidates from the Enamine library. The data sets at $i = 1$, $i = 5$ and $i = 9$ (Figure 7, upper panel) show the screening results from novel drug candidates generated by the Pareto-refined machine learning models after 1, 5 or 9 iteration cycles (Figure 6).

These best pH-specific candidates are found within the Pareto set of the screening results after each iteration, respectively. The 100 most pH-specific drug candidates from the Pareto set of the Enamine library (i = 0), after one optimization cycle (I = 1) and after nine (i = 9) optimization cycles are shown (Figure 7, lower panel).

## REFERENCES

[1] A. Thies et al. Modelling altered signalling of G-protein coupled receptors in inflamed environment to advance drug design. Scientific Reports, 13(607), 2023.

[2] C. Gorgulla et al. Virtual Flow 2.0 - The Next Generation Drug Discovery Platform Enabling Adaptive Screens of 69 Billion Molecules. bioRxiv, 2023.

[3] C. Schütte et al. Overcoming the timescale barrier in molecular dynamics: Transfer operators, variational principles and machine learning. Acta Numerica, 32:517-673, 2023.

[4] C. Secker et al. Novel multi-objective affinity approach allows to identify pH-specific $\mu$-opioid receptor agonists. 2023. under review.

[5] S. Ray et al. Coarse-Grained MD Simulations of Opioid Interactions with the $\mu$-Opioid Receptor and the Surrounding Lipid Membrane. Biophysica, 3(2):263 - 275, 2023.

[6] B. Schroeder et al. Nanoparticular inhibitors of flavivirus proteases from zika, west nile and dengue virus are cell-permeable antivirals. ACS Medicinal Chemistry Letters, 12(12):1955-1961,2021.

[7] M. H: Hofmann et al. BI-3406, a Potent and Selective SOS1-KRAS Interaction Inhibitor, Is Effective in KRAS-Driven Cancers through Combined MEK Inhibition. Cancer Discovery, 11(1):142-157, 01 2021.

[8] T. Blaschke et al. Reinvent 2.0: An AI tool for de novo drug design. Journal of Chemical Information and Modeling, 60(12):5918-5922, 2020. PMID: 33118816.

[9] J. Arùs-Pous et al. Randomized SMILES strings improve the quality of molecular generative models. J. Cheminform., 11(1):71, November 2019.

[10] R. Hillig et al. Discovery of potent SOS1 inhibitors that block RAS activation via disruption of the RAS-SOS1 interaction. Proc. Natl. Acad. Sci. U. S. A., 116(7):2551-2560, February 2019.

[11] D. Xenides et al. A metric space approach on the molecular vs. chemical similarity of some analgesic and euphoric compounds. January 2019.

[12] E. L. Wong et al. The transcription factor STAT5 catalyzes mannich ligation reactions yielding inhibitors of leukemic cell proliferation. Nat. Commun., 10(1):66, January 2019.

[13] A. Nigam et al. Augmenting genetic algorithms with deep neural networks for exploring the chemical space. CoRR, abs/1909.11655, 2019.

[14] B. Sanchez-Lengeling et al. Inverse molecular design using machine learning: Generative models for matter engineering. Science, 361(6400):360-365, 2018.

[15] V. Spahn et al. A nontoxic pain killer designed by modeling of pathological receptor conformations. Science, 355(6328):966 - 969, 2017.

[16] P. Kirkpatrick, C. Ellis, Chemical space. Nature 432, 823, 15.12.2004 (2004).

[17] J.-L. Reymond, The Chemical Space Project, Acc. Chem. Res. 2015, 48, 3, 722-730, February 17, 2015, https://doi.org/10.1021/ar500432k

[18] Lipinski, C. A.; Lombardo, F.; Dominy, B. W.; Feeney, P. J. Experimental and computational approaches to estimate solubility and permeability in drug discovery and development settings. Adv. Drug Delivery Rev.1997,23,3-25.(9)

[19] Bohacek, R. S.; McMartin, C.; Guida, W. C. The art and practice of structure-based drug design: A molecular modeling perspective. Med. Res. Rev.1996,16,3-50.

[20] D. Weininger SMILES, a chemical language and information system. Introduction to methodology and encoding rules, J. Chem. Inf. Comput. Sci. 1988, 28, 1, 31-36, February 1, 1988 https://doi.org/10.1021/ci00057a005.

[21] Gómez-Bombarelli, R., Wei, J. N., Duvenaud, D., Hernández-Lobato, J. M., Sánchez-Lengeling, B., Sheberla, D., Aguilera-Iparraguirre, J., Hirzel, T. D., Adams, R. P. and Aspuru-Guzik, A., Automatic Chemical Design Using a Data-Driven Continuous Representation of Molecules; ACS Central Science 2018 4 (2), 268-276 DOI: 10.1021/acscentsci.7b00572.

[22] Nigam, Akshat Kumar and Pollice, Robert and Krenn, Mario and Gomes, Gabriel dos Passos and Aspuru-Guzik, Alán; Beyond generative models: superfast traversal, optimization, novelty, exploration and discovery (STONED) algorithm for molecules using SELFIES, Chem. Sci., 2021, vol. 12, 20, 7079-7090, The Royal Society of Chemistry, doi 10.1039/D1SC00231G.

[23] Van Deursen, R. and Reymond, J.-L. (2007), Chemical Space Travel. ChemMedChem, 2: 636-640. https://doi.org/10.1002/cmdc.200700021.

**Claims**

1. A computer implemented method for identifying one or more ligands of interest for a target molecule, comprising:

   a. Providing a library comprising a plurality of (in silico) ligand structures,
   b. Calculating/generating one or more additional (in silico) ligand structure(s) based on the library, by employing a machine learning software (artificial intelligence; AI),
   c. Analyzing and/or evaluating one or more ligand structure(s) provided in a. and/or b. by determining one or more characteristics of said one or more ligand structure(s), comprising at least the binding characteristics of said one or more ligand structure(s) to the target molecule,
   d. Selecting one or more ligand structure(s) belonging to a Pareto set of the optimal one or more characteristics determined in c.,
   e. Repeating b.-d. at least once, thereby considering the library according to a. and/or the one or more ligand structure(s) belonging to the Pareto set selected in any previous iteration of d.,

   thereby determining the Pareto set of ligand structures for the one or more ligands of interest for a target molecule.

2. The method according to claim 1, wherein step d. further comprises adding the one or more ligand structure(s) belonging to the Pareto set either to the library provided in a. or providing said one or more structure(s) instead of the library in a.

3. The method according to any one of the preceding claims, wherein b.-d. are repeated according to e. until all structures additionally generated in b. belong to the Pareto set.

4. The method according to any one of the preceding claims, wherein the one or more characteristics determined in step c. further comprise one or more of target molecule (receptor) configuration, bioavailability (e.g., water solubility, fat solubility, solvent solubility), synthesizability, molecular weight and logP (hydrophilicity).

5. The method according to any one of the preceding claims, wherein the ligand structures are selected from simplified molecular input line entry specification (SMILES) strings, smiles arbitrary target specification (SMARTS) strings, Wiswesser Line Notation (WLN), ROSDAL, SLN and InChl, or any variation thereof.

6. The method according to any one of the preceding claims, wherein the ligand of interest is able to bind to an active center, a target biding site and/or an epitope binding site of the target molecule.

7. The method according to any one of the preceding claims, wherein the machine learning software (artificial intelligence; AI) is trained in advance based on a library comprising known chemical structures, preferably pharmaceutical compounds, signaling pathway members and/or receptor (ant-)agonists.

8. The method according to any one of the preceding claims, wherein the ligand of interest is a small molecule compound and/or wherein the library provided in a. comprises one or more (drug-like) small molecule compounds.

9. The method according to any one of the preceding claims, wherein a generated additional (*in silico*) ligand structure possesses a (pre-)defined chemical distance to the chemical structure it is generated from or to the previously generated ligand structure, such that said ligand structures differ in their chemical structures from each other by a (pre-)defined degree.

10. The method according to any one of the preceding claims, wherein the machine learning software or AI comprises a generative model, a generative adversarial network (GAN) or a deep generative model (DGM).

11. The method according to any one of the preceding claims, wherein the calculation/generation of the one or more additional ligand structure(s) in b. also comprises interpolation between known ligand structures from the library and/or the one or more ligand structure(s) belonging to the Pareto set provided instead of the library in a.

12. The method according any one of the preceding claims, wherein determining one or more characteristics of said one or more ligand structure(s) in c. comprises the employment of one or more computational (algorithmic) calculations for the ligand structures calculated/generated in b., preferably performed or supervised by a subject.

13. A software or computer program product for identifying one or more ligands of interest for a target molecule, wherein when said software or computer program product is executed the following steps are conducted:

a. Receiving data of a library comprising a plurality of (in silico) ligand structures,
b. Calculating/generating one or more additional (in silico) ligand structure(s) based on the library, by employing a machine learning software (artificial intelligence; AI),
c. Analyzing and/or evaluating one or more ligand structure(s) provided in a. and/or b. by determining one or more characteristics of said one or more ligand structure(s), comprising at least the binding characteristics of said one or more ligand structure(s) to a target molecule, and
d. Selecting one or more ligand structure(s) belonging to a Pareto set of the optimal one or more characteristics determined in c.,
e. Repeating b.-c. at least once, thereby considering the library according to a. and/or the one or more ligand structure(s) belonging to the Pareto set selected in any previous iteration of d., and thereby determining the Pareto set of ligand structures for the one or more ligands of interest,
f. optionally transmitting and displaying an output of the software or computer program product to a graphical user interface, preferably comprising the ligand structure(s) belonging to the Pareto set of ligand structures for the one or more ligands of interest.

14. A computer-readable storage device, comprising a software or computer program product according to the preceding claim.

**Fig. 1**

**A.**

Docking score vs. QED

**Fig. 1 continued**

**B.**

**Fig. 2**

**Fig. 3**

**Fig. 4**

ChEMBL Database → 1.5 mio. Molecules (SMILES) → Machine Learning on New Model → Pre-trained Model (Prior)

**Fig. 5**

Fig. 6

**Fig. 7**

**Fig. 7 continued**

$i = 5$    $i = 9$

$|\Delta F_P$ (neutral pH)

Europäisches
Patentamt

European
Patent Office

Office européen
des brevets

**EUROPEAN SEARCH REPORT**

**Application Number**

EP 24 16 2096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOITREAUD JACQUES ET AL: "OptiMol: Optimization of Binding Affinities in Chemical Space for Drug Discovery", JOURNAL OF CHEMICAL INFORMATION AND MODELING, vol. 60, no. 12, 28 September 2020 (2020-09-28), pages 5658-5666, XP055965390, US ISSN: 1549-9596, DOI: 10.1021/acs.jcim.0c00833 Retrieved from the Internet: URL:https://pubs.acs.org/doi/pdf/10.1021/acs.jcim.0c00833> | 1-8,10, 12-14 | INV. G16B15/30 G16C20/50 G16C20/70 G16C20/64 |
| Y | * p. 5658, Abstract p. 5658, right column, last p. 5660, left column, 2 p. 5661, Figure 2 and description thereof p. 5662, left column, 2 p. 5564, left column, 4 p. 5663, right column, last p. 5670, left column, 1 * | 9,11 | |

-/--

TECHNICAL FIELDS
SEARCHED      (IPC)

G16B
G16C

The present search report has been drawn up for all claims

| Place of search | Date of completion of the search | Examiner |
|---|---|---|
| Munich | 31 July 2024 | Mühlbauer, Max |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.........................................................................................
& : member of the same patent family, corresponding
   document

EPO FORM 1503 03.82 (P04C01)

page 1 of 3

Europäisches
Patentamt

European
Patent Office

Office européen
des brevets

**EUROPEAN SEARCH REPORT**

**Application Number**

EP 24 16 2096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & Boitreaud Jacques ET AL: "Supporting Information - OptiMol: Optimization of Binding Affinities in Chemical Space for Drug Discovery", Journal of Chemical Information and Modeling, vol. 60, no. 12, 28 September 2020 (2020-09-28), pages 1-13, XP093191136, US ISSN: 1549-9596, DOI: 10.1021/acs.jcim.0c00833 Retrieved from the Internet: URL:https://pubs.acs.org/doi/suppl/10.1021 /acs.jcim.0c00833/suppl_file/ci0c00833_si_ 001.pdf> * Supplementary Information, p. 11, Figure S7 * | | |
| Y,D | RAFAEL GÓMEZ-BOMBARELLI ET AL: "Automatic Chemical Design Using a Data-Driven Continuous Representation of Molecules", ACS CENTRAL SCIENCE, vol. 4, no. 2, 12 January 2018 (2018-01-12), pages 268-276, XP055589835, ISSN: 2374-7943, DOI: 10.1021/acscentsci.7b00572 | 9,11 | TECHNICAL FIELDS SEARCHED (IPC) |
| A | * whole document, in particular p. 270, Figure 2 and description thereof * | 1-8,10, 12-14 | |

-/--

The present search report has been drawn up for all claims

| Place of search | Date of completion of the search | Examiner |
|---|---|---|
| Munich | 31 July 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

**Application Number**

EP 24 16 2096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FROMER JENNA C. ET AL: "Computer-aided multi-objective optimization in small molecule discovery", PATTERNS, vol. 4, no. 2, 1 February 2023 (2023-02-01), page 100678, XP093191088, ISSN: 2666-3899, DOI: 10.1016/j.patter.2023.100678 * the whole document * | 1-14 | |
| A | SECKER CHRISTOPHER ET AL: "Novel multi-objective affinity approach allows to identify pH-specific [mu]-opioid receptor agonists", JOURNAL OF CHEMINFORMATICS, vol. 15, no. 1, 19 September 2023 (2023-09-19), XP093190561, London, UK ISSN: 1758-2946, DOI: 10.1186/s13321-023-00746-4 Retrieved from the Internet: URL:https://link.springer.com/article/10.1186/s13321-023-00746-4/fulltext.html> * the whole document * | 1-14 | |
| A | Gorgulla Christoph: "Structure-Based Ultra-Large Virtual Screenings : Methods and Applications - Part V Structure-Based Virtual Screening Using Docking" In: "Computational Drug Discovery : Methods and Applications", 19 January 2024 (2024-01-19), Wiley, XP093190898, pages 441-470, DOI: 10.1002/9783527840748.ch19, * the whole document * | 1-14 | TECHNICAL FIELDS SEARCHED (IPC) |

The present search report has been drawn up for all claims

| Place of search | Date of completion of the search | Examiner |
|---|---|---|
| Munich | 31 July 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. THIES et al.** Modelling altered signalling of G-protein coupled receptors in inflamed environment to advance drug design.. *Scientific Reports*, 2023, vol. 13 (607)
- **C. GORGULLA et al.** Virtual Flow 2.0 - The Next Generation Drug Discovery Platform Enabling Adaptive Screens of 69 Billion Molecules.. *bioRxiv*, 2023
- **C. SCHÜTTE et al.** Overcoming the timescale barrier in molecular dynamics: Transfer operators, variational principles and machine learning.. *Acta Numerica*, 2023, vol. 32, 517-673
- **C. SECKER et al.** *Novel multi-objective affinity approach allows to identify pH-specific $\mu$-opioid receptor agonists*, 2023
- **S. RAY et al.** Coarse-Grained MD Simulations of Opioid Interactions with the $\mu$-Opioid Receptor and the Surrounding Lipid Membrane. *Biophysica*, 2023, vol. 3 (2), 263-275
- **B. SCHROEDER et al.** Nanoparticular inhibitors of flavivirus proteases from zika, west nile and dengue virus are cell-permeable antivirals.. *ACS Medicinal Chemistry Letters*, 2021, vol. 12 (12), 1955-1961
- **M. H: HOFMANN et al.** BI-3406, a Potent and Selective SOS1-KRAS Interaction Inhibitor, Is Effective in KRAS-Driven Cancers through Combined MEK Inhibition. *Cancer Discovery*, January 2021, vol. 11 (1), 142-157
- **T. BLASCHKE et al.** Reinvent 2.0: An AI tool for de novo drug design. *Journal of Chemical Information and Modeling*, 2020, vol. 60 (12), 5918-5922
- **J. ARÙS-POUS et al.** Randomized SMILES strings improve the quality of molecular generative models.. *J. Cheminform.*, November 2019, vol. 11 (1), 71
- **R. HILLIG et al.** Discovery of potent SOS1 inhibitors that block RAS activation via disruption of the RAS-SOS1 interaction. *Proc. Natl. Acad. Sci. U. S. A.*, February 2019, vol. 116 (7), 2551-2560
- **D. XENIDES et al.** *A metric space approach on the molecular vs. chemical similarity of some analgesic and euphoric compounds.*, January 2019
- **E. L. WONG et al.** The transcription factor STAT5 catalyzes mannich ligation reactions yielding inhibitors of leukemic cell proliferation. *Nat. Commun.*, January 2019, vol. 10 (1), 66

- **A. NIGAM et al.** Augmenting genetic algorithms with deep neural networks for exploring the chemical space. *CoRR*, 2019
- **B. SANCHEZ-LENGELING et al.** Inverse molecular design using machine learning: Generative models for matter engineering.. *Science*, 2018, vol. 361 (6400), 360-365
- **V. SPAHN et al.** A nontoxic pain killer designed by modeling of pathological receptor conformations.. *Science*, 2017, vol. 355 (6328), 966-969
- **P. KIRKPATRICK** ; **C. ELLIS**. Chemical space. *Nature*, 15 December 2004, vol. 432, 823
- **J.-L. REYMOND**. The Chemical Space Project. *Acc. Chem. Res.*, 17 February 2015, vol. 48 (3), 722-730, https://doi.org/10.1021/ar500432k
- **LIPINSKI, C. A.** ; **LOMBARDO, F** ; **DOMINY, B. W** ; **FEENEY, P. J.** Experimental and computational approaches to estimate solubility and permeability in drug discovery and development settings. *Adv. Drug Delivery Rev.*, 1997, vol. 23 (9), 3-25
- **BOHACEK, R. S** ; **MCMARTIN, C** ; **GUIDA, W. C**. The art and practice of structure-based drug design: A molecular modeling perspective. *Med. Res. Rev.*, 1996, vol. 16, 3-50
- **D. WEININGER**. SMILES, a chemical language and information system. Introduction to methodology and encoding rules. *J. Chem. Inf. Comput. Sci.*, 01 February 1988, vol. 28 (1), 31-36, https://doi.org/10.1021/ci00057a005
- **GÓMEZ-BOMBARELLI, R** ; **WEI, J. N.** ; **DUVENAUD, D** ; **HERNÁNDEZ-LOBATO, J. M.** ; **SÁNCHEZ-LENGELING, B.** ; **SHEBERLA, D.** ; **AGUILERA-LPARRAGUIRRE, J.** ; **HIRZEL, T. D.** ; **ADAMS, R. P.** ; **ASPURU-GUZIK, A**. Automatic Chemical Design Using a Data-Driven Continuous Representation of Molecules. *ACS Central Science*, 2018, vol. 4 (2), 268-276
- **NIGAM, AKSHAT KUMAR** ; **POLLICE, ROBERT** ; **KRENN, MARIO** ; **GOMES, GABRIEL DOS PASSOS** ; **ASPURU-GUZIK, ALÁN**. Beyond generative models: superfast traversal, optimization, novelty, exploration and discovery (STONED) algorithm for molecules using SELFIES, Chem. Sci. *The Royal Society of Chemistry*, 2021, vol. 12 (20), 7079-7090
- **VAN DEURSEN, R.** ; **REYMOND, J.-L.** Chemical Space Travel. *ChemMedChem*, 2007, vol. 2, 636-640, https://doi.org/10.1002/cmdc.200700021